(19)
**Europäisches Patentamt**
**European Patent Office**
**Office européen des brevets**

(11) **EP 3 244 743 B1**

(12) **EUROPEAN PATENT SPECIFICATION**

(45) Date of publication and mention
of the grant of the patent:
**23.10.2019 Bulletin 2019/43**

(51) Int Cl.:
*A21D 2/26* *(2006.01)*     *A21D 8/04* *(2006.01)*
*C12N 9/10* *(2006.01)*     *A21D 13/40* *(2017.01)*
*A21D 10/00* *(2006.01)*

(21) Application number: **16700753.3**

(22) Date of filing: **15.01.2016**

(86) International application number:
**PCT/EP2016/050788**

(87) International publication number:
**WO 2016/113399 (21.07.2016 Gazette 2016/29)**

(54) **METHOD TO IMPROVE SLICEABILITY OF BAKED GOODS**

VERFAHREN ZUR VERBESSERUNG DER SCHNEIDBARKEIT VON BACKWAREN

PROCÉDÉ POUR AMÉLIORER LE TRANCHAGE DE PRODUITS DE BOULANGERIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.01.2015 EP 15151444**

(43) Date of publication of application:
**22.11.2017 Bulletin 2017/47**

(73) Proprietor: **Novozymes A/S
2880 Bagsvaerd (DK)**

(72) Inventors:
• **BELLIDO, Guillermo
4243 Dittingen (CH)**

• **SCHOENENBERG, Sven
4243 Dittingen (CH)**

(74) Representative: **NZ EPO Representatives
Krogshoejvej 36
2880 Bagsvaerd (DK)**

(56) References cited:
**EP-A1- 0 412 607     EP-A1- 0 691 407
EP-A1- 1 559 788     WO-A1-00/58445
WO-A1-96/33267     WO-A1-97/41736
WO-A1-98/13479     WO-A1-2012/010592**

EP 3 244 743 B1

## Description

### Reference to a sequence listing

This application contains a Sequence Listing in computer readable form.

### Background of the invention

### Field of the invention

The present invention relates to a method of improving the sliceability of baked goods, e.g., bread, by using a glycosyltransferase, a cyclomaltodextrin glucanotransferase, or a mixture of a glycosyltransferase and a cyclomaltodextrin glucanotransferase.

### Description of the related art

In the manufacturing of baked goods, particularly in high speed bread production or in industrialized processes using automated or semi-automated equipment, slicing is an important unit process operation that follows baking and precedes packing.

Bread, e.g., toast and sandwich bread, is typically cooled before slicing and packing, otherwise, the crumb will have poor sliceability properties. On removal from the oven, bread has a temperature of around 150-180°C at its crust and 95-100°C in its core.

During the cooling process, the baked bread loses moisture and the gelatinized starch sets into the final crumb structure. Setting of the crumb leads to the firming of the bread which in turn facilitates slicing.

Depending on the size and shape of the loaf, it may take up to 2-3 hours for the bread to completely cool and at this point the temperature gradient between the crust and the crumb becomes zero. However, in high speed bread production slicing of the bread is accomplished before bread is completely cooled. This is critical, as unwarranted prolongation of the cooling process causes, among others, undue extension of the manufacturing process, excessive loss of moisture, and bigger risk of microbial contamination.

In the manufacturing of baked goods, particularly in high speed bread production, the end of the cooling cycle is reached when the core temperature of bread is reduced to a desirable temperature range, most commonly 30-38°C. If bread is sliced at a higher temperature range, the crumb is typically gummy and sticks to the cutting blades, causing quality issues or production issues, including production downtime, such as to clean the blades of the slicer to avoid damaging of bread slices.

In the manufacturing of baked goods, especially in high speed bread production, cooling of bread is accelerated by using various types of cooling systems, e.g., convection cooling, conditioned air cooling, and vacuum cooling, each of which offers different benefits in terms of degree of control of moisture loss in the cooling loaf, speed of the cooling process, energy consumption and capital expense among other considerations. There are situations in which slicing of bread at temperatures higher than 30-38°C is desirable, e.g., slicing of bread at a temperature in the range of from 38°C to 55°C.

Some of the advantages of slicing bread at higher temperatures include higher processing throughputs, shorter overall processing times (due to shorter cooling times), energy savings due to the need to remove less heat from the cooling bread, and improved process robustness as slicing can be accomplished over a wider temperature range. However, slicing bread at the desirable temperature range of 30-38°C or a customarily lower temperature range may still cause sliceability issues. Such issues may arise due to the need to change bread formulation (e.g., new flour batch, flour from a new crop year, addition/removal of bread additives, addition/removal of processing aids, and addition/removal of new functional ingredients), bread manufacturing practices, and introduction of new baking equipment technology or other changes in bread production.

Bread rejects due to slicing defects can include such defects as the permanent deformation of single or multiple slices, deformation of the whole loaf, sticking together of two or more contiguous slices, loosening or peeling off of crumb surface, excessive removal of crumbs from crumb surface, any undue deterioration of crumb grain texture (e.g., crumb holes), or more than one of the aforementioned defects combined.

Recently, the product Soft'r Intens Sliceability™ (produced by Puratos) has been commercially available.

There is still a need in the art for improving the slicing properties of toast bread, especially soft toast bread, when sliced at high core temperatures.

## Summary of the invention

The inventors have found that the addition to the dough of a glycosyltransferase and/or a cyclomaltodextrin glucanotransferase has a surprising effect on the sliceability of the baked product when slicing the baked product at a high core temperature.

Accordingly, the invention provides a method of improving sliceability of a baked product prepared from dough comprising incorporating into the dough a glycosyltransferase and/or a cyclomaltodextrin glucanotransferase; and slicing the baked product at a core temperature of the baked product of 30-55°C.

In particular, the invention provides a method of improving sliceability of a baked product prepared from dough comprising:

- incorporating into the dough a glycosyltransferase (EC 2.4.1.18) and/or a cyclomaltodextrin glucanotransferase (EC 2.4.1.19);
- baking the dough into a baked product; and
- slicing the baked product during the cooling period when the baked product has a core temperature of 30-55°C.

In one embodiment, the glycosyltransferase has at least 80% sequence identity to the polypeptide of SEQ ID NO: 1.

In one embodiment, the cyclomaltodextrin glucanotransferase has at least 80% sequence identity to the polypeptide of SEQ ID NO: 2.

In one embodiment, a glycosyltransferase and a cyclomaltodextrin glucanotransferase are incorporated into the dough.

In one embodiment, additionally an enzyme selected from the group consisting of amylase, xylanase, glucanase, galactanase, mannanase, aminopeptidase, alpha-amylase, beta-amylase, anti-staling alpha-amylase, carboxypeptidase, catalase, chitinase, cutinase, deoxyribonuclease, esterase, alpha-galactosidase, beta-galactosidase, glucoamylase, alpha-glucosidase, beta-glucosidase, haloperoxidase, invertase, laccase, lipase, phospholipase, mannosidase, oxidase, pectinolytic enzymes, peptidoglutaminase, protease, peroxidase, phytase, and polyphenoloxidase is added to the dough.

In one embodiment, the glycosyltransferase and/or the the cyclomaltodextrin glucanotransferase is applied in an amount of 0.01-100 mg enzyme protein per kg flour.

In one embodiment, the baked product is sliced at a core temperature of the baked product of 38-55°C.

The sliceability of the baked product is improved compared to a baked product prepared from dough wherein no glycosyltransferase and/or a cyclomaltodextrin glucanotransferase is added to the dough.

In one embodiment, the crumb structure of the baked product is improved compared to a baked product prepared from dough wherein no glycosyltransferase and/or a cyclomaltodextrin glucanotransferase is added to the dough.

In one embodiment, the baked product is a bread; preferably a toast bread; in particular a lidded toast bread.

Further disclosed is a baking composition comprising a glycosyltransferase and/or a cyclomaltodextrin glucanotransferase for use in improving sliceability in a baked product.

In one embodiment, the baking composition further comprises an enzyme selected from the group consisting of amylase, glucanase, galactanase, mannanase, aminopeptidase, alpha-amylase, beta-amylase, anti-staling alpha-amylase, carboxypeptidase, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, mannosidase, oxidase, pectinolytic enzymes, peptidoglutaminase, protease, peroxidase, phytase, and polyphenoloxidase.

Further disclosed is a pre-mix comprising a baking composition comprising a glycosyltransferase and/or a cyclomaltodextrin glucanotransferase, flour, and one or more dough or bread additives for use in improving sliceability in a baked product.

Further disclosed is the use of a baking composition comprising a glycosyltransferase and/or a cyclomaltodextrin glucanotransferase for improving sliceability of a baked product, wherein the baked product has a core temperature of 30-55°C.

## Brief description of the figures

FIG 1: Sliceability with a mixture of glycosyltransferase (SEQ ID NO:1) and cyclomaltodextrin glucanotransferase (SEQ ID NO:2).

FIG 2: Sliceability with glycosyltransferase (SEQ ID NO:1).

FIG 3: Sliceability with cyclomaltodextrin glucanotransferase (SEQ ID NO:2).

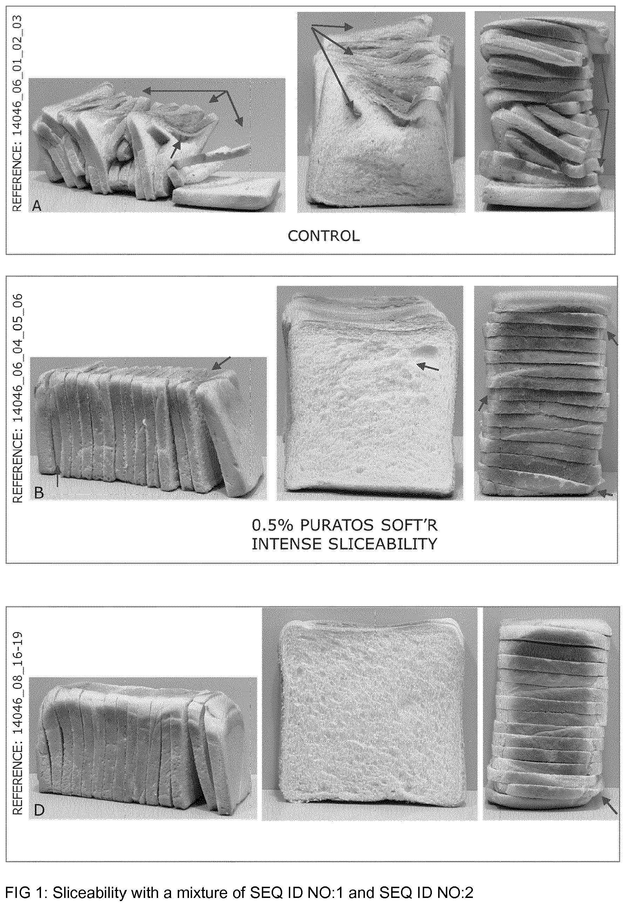

FIG 1-3: The breads were made according to Example 2-4 and sliced at a core temperature of 48-50°C and a slice thickness of 12.5 mm.

**Definitions**

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Fragment:** The term "fragment" means a polypeptide having one or more (*e.g.* several) amino acids absent from the amino and/or carboxyl terminus of a mature polypeptide or domain.

**Host cell**: The term "host cell" means any cell type that is susceptible to transformation, transfection, transduction, or the like with a nucleic acid construct or expression vector comprising a polynucleotide of the present disclosure. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.* multiple copies of a gene encoding the substance; use of a stronger promoter than the promoter naturally associated with the gene encoding the substance). An isolated substance may be present in a fermentation broth sample.

**Mature polypeptide:** The term "mature polypeptide" means a polypeptide in its final form following translation and any post-translational modifications, such as N-terminal processing, C-terminal truncation, glycosylation, phosphorylation, etc.

**Mature polypeptide coding sequence:** The term "mature polypeptide coding sequence" means a polynucleotide that encodes a mature a glycosyltransferase or a cyclomaltodextrin glucanotransferase polypeptide.

**Sequence identity:** The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues x 100})/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides x 100})/(\text{Length of Alignment} - \text{Total Number of Gaps in Alignment}).$$

**Improved property:** When incorporated into a dough in effective amounts, the glycosyltransferase and/or a cyclomaltodextrin glucanotransferase, may improve one or more properties of the baked product obtained therefrom relative to a baked product in which the glycosyltransferase and/or a cyclomaltodextrin glucanotransferase are not incorporated.

The term "improved property" is defined herein as any property of a baked product, which is improved by the action of a glycosyltransferase and/or a cyclomaltodextrin glucanotransferase relative to a baked product in which a glycosyltransferase and/or a cyclomaltodextrin glucanotransferase employed in the invention is not incorporated.

The improved property may include, but is not limited to, improved sliceability of the baked product, improved flavor of the baked product, improved crumb structure of the baked product, improved crumb surface of the baked product, and/or improved texture of the baked product.

The improved property may be determined by comparison of a baked product prepared with and without addition of a glycosyltransferase and/or a cyclomaltodextrin glucanotransferase in accordance with the methods of the present invention which are described below.

**Improved flavor of the baked product:** Organoleptic qualities may be evaluated using procedures well established in the baking industry, and may include, for example, the use of a panel of trained taste-testers.

**Improved crumb structure of the baked product:** The term "improved crumb structure of the baked product" is defined herein as the property of a baked product with finer cells and/or thinner cell walls in the crumb and/or more uniform/homogenous distribution of cells in the crumb and is usually evaluated visually by the baker or by digital image analysis as known in the art (e.g. C-cell, Calibre Control International Ltd, Appleton, Warrington, UK).

**Improved sliceability:** While slicing is a common unit process operation in the manufacturing of many baked goods, no standard method exists to measure bread sliceability. According to the present invention, improved sliceability is defined as the degree to which baked bread having a given core temperature can be sliced into slices with fewer defects - compared to a control - using a slicing machine or a hand knife and a standardized protocol.

## Detailed description of the invention

The invention provides a method for preparing a baked product prepared from the dough which method comprises incorporating into the dough a glycosyltransferase and/or a cyclomaltodextrin glucanotransferase.

The disclosure also provides baking compositions, and pre-mix comprising a glycosyltransferase and/or a cyclomaltodextrin glucanotransferase.

## Glycosyltransferases

Glycosyltransferases are enzymes (EC 2.4.1.18) that establish natural glycosidic linkages, including the biosynthesis of disaccharides, oligosaccharides and polysaccharides. They catalyse the transfer of monosaccharide moieties from activated nucleotide sugar (also known as the "glycosyl donor") to a glycosyl acceptor molecule, usually an alcohol.

Glycosyltransferase activity may be determined as known in the art, e.g., by using a modified version of the procedure described by Takata et al., Applied and Environmental Microbiology (1994), p. 3097 (assay A). Fifty microliter enzyme solution is diluted in double-deionized water and mixed with 50 microliter substrate solution and incubated for 30 min at 50°C. The substrate solution is 0.075% type III amylose dissolved in 78 mM Tris buffer (e.g., 30 mg amylose dissolved by subsequent addition of 0.8 mL 96% ethanol, 2 mL 2 M NaOH, 4 mL double de-ionized water and 2 mL 2M HCl. Finally 31.2 mL 0.1 M Tris buffer (pH 7.2 at 22°C)). The reaction is terminated by the addition of 150 microliter of iodine reagent. Iodine reagent is made daily from 0.5 mL of stock solution (0.26 g of $I_2$ and 2.6 g of KI in 10 mL of double de-ionized water) mixed with 0.5 mL of 1 M HCl, and diluted to a total volume of 130 mL. The mixture is incubated for 5 minutes at 50 °C to form the color. Activity is measured as difference in absorbance at 660 nm between a tested sample and a control in which cell extract (enzyme solution) is replaced by double de-ionized water. One unit of branching enzyme activity is defined as the amount of enzyme that can decrease the absorbance at 660 nm compared to the control by 0.7% per minute at 50°C under the conditions described above.

Useful glycosyltransferases employed in the present invention are described in US 2007/0015679. In particular, a glycosyltransferase to be applied in the methods of the present invention, relates to isolated polypeptides comprising amino acid sequences having a degree of sequence identity to the polypeptide of SEQ ID NO: 1 of preferably at least 80%, e.g., at least 81%, e.g., at least 82%, e.g., at least 83%, e.g., at least 84%, e.g., at least 85%, e.g., at least 86%, e.g., at least 87%, e.g., at least 88%, e.g., at least 89%, e.g., at least 90%, e.g., at least 91%, e.g., at least 92%, e.g., at least 93%, e.g., at least 94%, e.g., at least 95%, e.g., at least 96%, e.g., at least 97%, e.g., at least 98%, e.g., at least 99%, e.g., 100%, which have glycosyltransferase activity.

In a preferred aspect, the glycosyltransferase employed in the invention comprise amino acid sequences that differ by 1-10 amino acids, e.g., by up to ten amino acids, e.g., by up to nine amino acids, e.g., by up to eight amino acids, preferably by up to seven amino acids, e.g., by up to six amino acids, e.g., by up to five amino acids, preferably by up to four amino acids, e.g., by up to three amino acids, e.g., by up to two amino acids, and e.g., by one amino acid from the polypeptide of SEQ ID NO: 1.

A glycosyltransferase preferably comprises the amino acid sequence of SEQ ID NO: 1 or an allelic variant thereof; or a fragment thereof having glycosyltransferase activity.

In one embodiment, the glycosyltransferase comprises the amino acid sequence of SEQ ID NO: 1. In one embodiment, the glycosyltransferase consists of the amino acid sequence of SEQ ID NO: 1.

The amino acid sequence of SEQ ID NO: 1; or a fragment thereof, may be used to design nucleic acid probes to identify and clone DNA encoding polypeptides having glycosyltransferase activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 14, preferably at least 25, more preferably at least 35, and most preferably at least 70 nucleotides in length. Preferably, the nucleic acid probe is at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, e.g., nucleic acid probes that are preferably at least 600 nucleotides, more preferably at least 700 nucleotides, even more preferably at least 800 nucleotides, or most preferably at least 900 nucleotides in length. Both DNA and RNA probes can be used. The probes are typically labeled for detecting the corresponding gene (for example, with $^{32}$P, $^{3}$H, $^{35}$S, biotin, or avidin).

A genomic DNA or cDNA library prepared from such other strains may, therefore, be screened for DNA that hybridizes with the probes described above and encodes a glycosyltransferase. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material.

For purposes of the present invention, hybridization indicates that the nucleotide sequence hybridizes to a labeled nucleic acid probe corresponding to the polypeptide coding sequence of SEQ ID NO: 1.

In another aspect, the glycosyltransferase comprises a substitution, deletion, and/or insertion of one or more (or several) amino acids of the polypeptide of SEQ ID NO: 1, or a homologous sequence thereof. Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for boosting effect on starch saccharification to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identities of essential amino acids can also be inferred from analysis of identities with polypeptides that are related to the parent polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The total number of amino acid substitutions, deletions and/or insertions of the mature polypeptide of SEQ ID NO: 1 may not be more than 10, *e.g.*, 1, 2, 3, 4, 5, 6, 7, 8 or 9.

A genomic DNA or cDNA library prepared from such other strains may, therefore, be screened for DNA that hybridizes with the probes described above and encodes a glycosyltransferase. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material.

For purposes of the present invention, hybridization indicates that the nucleotide sequence hybridizes to a labeled nucleic acid probe corresponding to the polypeptide coding sequence of SEQ ID NO: 1.

In another aspect, the glycosyltransferase comprises a substitution, deletion, and/or insertion of one or more (or several) amino acids of the polypeptide of SEQ ID NO: 1, or a homologous sequence thereof. Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

**Cyclomaltodextrin glucanotransferases**

A cyclomaltodextrin glucanotransferase (EC 2.4.1.19) is an enzyme that catalyzes the chemical reaction of cyclizing part of a 1,4-alpha-D-glucan molecule through the formation of a 1,4-alpha-D-glucosidic bond. It belongs to the family of glycosyltransferases.

Cyclomaltodextrin glucanotransferase activity may be measured as known in the art, e.g., by incubating enzyme with 5.92 g/L starch in 85 mM HEPES buffer (pH 6.2) and 0.46 mM $Ca^{2+}$ for 20 min at 50 °C. Subsequently, increase in reducing sugar may be determined by appropriate method (e.g. PAHBAH/Bi complexing). One enzyme unit is defined as the amount of enzyme forming 1 micromol of reducing sugar (glucose equivalents) per min at the conditions described above.

Useful cyclomaltodextrin glucanotransferases employed in the present invention are described in WO 89/03421. In particular, a cyclomaltodextrin glucanotransferase to be applied in the methods of the present invention, relates to isolated polypeptides comprising amino acid sequences having a degree of sequence identity to the polypeptide of SEQ ID NO: 2 of preferably at least 80%, e.g., at least 81%, e.g., at least 82%, e.g., at least 83%, e.g., at least 84%, e.g., at least 85%, e.g., at least 86%, e.g., at least 87%, e.g., at least 88%, e.g., at least 89%, e.g., at least 90%, e.g., at least 91%, e.g., at least 92%, e.g., at least 93%, e.g., at least 94%, e.g., at least 95%, e.g., at least 96%, e.g., at least 97%, e.g., at least 98%, e.g., at least 99%, e.g., 100%, which have cyclomaltodextrin glucanotransferase activity.

In a preferred aspect, the cyclomaltodextrin glucanotransferase employed in the invention comprise amino acid sequences that differ by 1-10 amino acids, preferably by up to ten amino acids, preferably by up to nine amino acids, preferably by up to eight amino acids, preferably by up to seven amino acids, preferably by up to six amino acids, preferably by up to five amino acids, preferably by up to four amino acids, preferably by up to three amino acids, preferably by up to two amino acids, and preferably by one amino acid from the polypeptide of SEQ ID NO: 2.

A cyclomaltodextrin glucanotransferase preferably comprises the amino acid sequence of SEQ ID NO: 2 or an allelic variant thereof; or a fragment thereof having cyclomaltodextrin glucanotransferase activity.

In one embodiment, the cyclomaltodextrin glucanotransferase comprises the amino acid sequence of SEQ ID NO: 2. In one embodiment, the cyclomaltodextrin glucanotransferase consists of the amino acid sequence of SEQ ID NO: 2.

The amino acid sequence of SEQ ID NO: 2; or a fragment thereof, may be used to design nucleic acid probes to identify and clone DNA encoding polypeptides having cyclomaltodextrin glucanotransferase activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 14, preferably at least 25, more preferably at least 35, and most preferably at least 70 nucleotides in length. Preferably, the nucleic acid probe is at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, e.g., nucleic acid probes that are preferably at least 600 nucleotides, more preferably at least 700 nucleotides, even more preferably at least 800 nucleotides, or most preferably at least 900 nucleotides in length. Both DNA and RNA probes can be used. The probes are typically labeled for detecting the corresponding gene (for example, with $^{32}$P, $^3$H, $^{35}$S, biotin, or avidin).

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for boosting effect on starch saccharification to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identities of essential amino acids can also be inferred from analysis of identities with polypeptides that are related to the parent polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The total number of amino acid substitutions, deletions and/or insertions of the mature polypeptide of SEQ ID NO: 2 may not be more than 10, *e.g.*, 1, 2, 3, 4, 5, 6, 7, 8 or 9.

A genomic DNA or cDNA library prepared from such other strains may, therefore, be screened for DNA that hybridizes with the probes described above and encodes a cyclomaltodextrin glucanotransferase. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material.

For purposes of the present invention, hybridization indicates that the nucleotide sequence hybridizes to a labeled nucleic acid probe corresponding to the polypeptide coding sequence of SEQ ID NO: 2.

In another aspect, the glycosyltransferase comprises a substitution, deletion, and/or insertion of one or more (or several) amino acids of the polypeptide of SEQ ID NO: 2, or a homologous sequence thereof. Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

**Baking compositions**

The present disclosure relates to baking compositions comprising a glycosyltransferase, a cyclomaltodextrin glucanotransferase, or a mixture of a glycosyltransferase and a cyclomaltodextrin glucanotransferase, and their preparation, e.g., compositions for improving the sliceability in a baked product.

The composition may further comprise one or more additional enzymes, in particular amylase, xylanase, glucanase, galactanase, mannanase, aminopeptidase, alpha-amylase, beta-amylase, anti-staling alpha-amylase, carboxypeptidase, catalase, chitinase, cutinase, deoxyribonuclease, esterase, alpha-galactosidase, beta-galactosidase, glucoamylase, alpha-glucosidase, beta-glucosidase, haloperoxidase, invertase, laccase, lipase, phospholipase, mannosidase, oxidase, pectinolytic enzymes, peptidoglutaminase, protease, peroxidase, phytase, and polyphenoloxidase.

The compositions may be prepared in accordance with methods known in the art and may have any physical appearance such as liquid, paste or solid. For instance, the composition may be formulated using methods known to the art of formulating enzymes and/or pharmaceutical products, e.g., into coated or uncoated granules or micro-granules. The glycosyltransferase, the cyclomaltodextrin glucanotransferase, or the mixture of a glycosyltransferase and a cyclomaltodextrin glucanotransferase, and any additional enzymes to be included in the composition may be stabilized in accordance with methods known in the art, e.g., by stabilizing the polypeptide in the composition by adding an antioxidant or reducing agent to limit oxidation of the polypeptide or it may be stabilized by adding polymers such as PVP, PVA, PEG, or other suitable polymers known to be beneficial to the stability of polypeptides in solid or liquid compositions.

When formulating the glycosyltransferase, the cyclomaltodextrin glucanotransferase, or the mixture of a glycosyltransferase and a cyclomaltodextrin glucanotransferase as a granulate or agglomerated powder, the particles preferably have a narrow particle size distribution with more than 95% (by weight) of the particles in the range from 25 to 500 μm. Granulates and agglomerated powders may be prepared by conventional methods, e.g., by spraying the glycosyltransferase, the cyclomaltodextrin glucanotransferase, or the mixture of a glycosyltransferase and a cyclomaltodextrin glucanotransferase onto a carrier in a fluid-bed granulator. The carrier may consist of particulate cores having a suitable particle size. The carrier may be soluble or insoluble, e.g., a salt (such as NaCl or sodium sulfate), a sugar (such as sucrose or lactose), a sugar alcohol (such as sorbitol), starch, rice, corn grits, or soy. The composition is preferably in the form of a dry powder or granulates, in particular a non-dusting granulate.

In one embodiment, the disclosure provides a granule comprising a glycosyltransferase, a cyclomaltodextrin glucanotransferase, or a mixture of a glycosyltransferase and a cyclomaltodextrin glucanotransferase.

In a particular embodiment, the composition is a dough composition or a dough improving additive or a premix comprising a glycosyltransferase, a cyclomaltodextrin glucanotransferase, or a mixture of a glycosyltransferase and a cyclomaltodextrin glucanotransferase.

The term "pre-mix" is defined herein to be understood in its conventional meaning, i.e., as a mix of baking agents, generally including flour, which may be used in industrial bread-baking plants, in retail bakeries, in baking mixes for home use, etc.

The pre-mix may be prepared by mixing the baking composition of the disclosure with a suitable carrier such as flour, starch, a sugar, a complex carbohydrate such as maltodextrin, or a salt. The pre-mix may contain other dough and/or bread additives, e.g., any of the additives, including enzymes, mentioned herein.

**The additional enzymes**

Optionally, additional enzymes, such as amylase, xylanase, glucanase, galactanase, mannanase, aminopeptidase, alpha-amylase, beta-amylase, anti-staling alpha-amylase, carboxypeptidase, catalase, chitinase, cutinase, deoxyribonuclease, esterase, alpha-galactosidase, beta-galactosidase, glucoamylase, alpha-glucosidase, beta-glucosidase, haloperoxidase, invertase, laccase, lipase, phospholipase, mannosidase, oxidase, pectinolytic enzymes, peptidoglutaminase, protease, peroxidase, phytase, and polyphenoloxidase may be used together with the glycosyltransferase, the cyclomaltodextrin glucanotransferase, or the mixture of a glycosyltransferase and a cyclomaltodextrin glucanotransferase.

The additional enzyme may be of any origin, including mammalian and plant, and preferably of microbial (bacterial, yeast or fungal) origin.

The glucoamylase for use in the present invention include the *A. niger* G1 or G2 glucoamylase (Boel et al. (1984), EMBO J. 3 (5), p. 1097-1102), or the *A. awamori* glucoamylase disclosed in WO 84/02921, or the *A. oryzae* glucoamylase (Agric. Biol. Chem. (1991), 55 (4), p. 941-949).

The amylase may be fungal or bacterial, e.g., a maltogenic alpha-amylase from *B. stearothermophilus* or an alpha-amylase from *Bacillus*, e.g., *B. licheniformis* or *B. amyloliquefaciens*, a beta-amylase, e.g., from plant (e.g. soy bean) or from microbial sources (e.g. *Bacillus*), or a fungal alpha-amylase, e.g. from *A. oryzae*.

Suitable commercial anti-staling alpha-amylases include NOVAMYL™, in particular Novamyl 10 000 BG™, Novamyl 1500 MG™, Novamyl L™, and Novamyl Pro 80 BG™, Novamyl Pro 12 BG™, Novamyl Sweet™, Novamyl 3D™, OptiCake 50 BG™ and OptiCake Fresh BG™ (available from Novozymes A/S).

An anti-staling amylase for use in the invention may also be an amylase (glucan 1,4-alpha-maltotetrahydrolase (EC 3.2.1.60)) from *Pseudomonas saccharophilia* or variants thereof, e.g., Powerfresh G3™ or Powerfresh G4™ (available from Dupont/Danisco).

Suitable commercial fungal alpha-amylase compositions include, e.g., BAKEZYME P 300™ (available from DSM) and FUNGAMYL 2500 BG™, FUNGAMYL 4000 BG™, FUNGAMYL 800 L™, FUNGAMYL ULTRA BG™ and FUNGAMYL ULTRA SG™ (available from Novozymes A/S).

The glucose oxidase may be a fungal glucose oxidase, in particular an *Aspergillus niger* glucose oxidase (such as GLUZYME™, available from Novozymes A/S, Denmark).

The protease may be from *Bacillus*, e.g., *B. amyloliquefaciens*.

The phospholipase may have phospholipase A1, A2, B, C, D or lysophospholipase activity; it may or may not have lipase activity. It may be of animal origin, e.g. from pancreas, snake venom or bee venom, or it may be of microbial origin, e.g. from filamentous fungi, yeast or bacteria, such as *Aspergillus or Fusarium, e.g. A. niger, A. oryzae* or *F. oxysporum.* A preferred lipase/phospholipase from *Fusarium oxysporum* is disclosed in WO 98/26057. Also, the variants described in WO 00/32758 may be used.

Suitable phospholipase compositions are LIPOPAN F™ and LIPOPAN XTRA™ (available from Novozymes A/S) or PANAMORE GOLDEN™ and PANAMORE SPRING™ (available from DSM).

The xylanase may be derived from a strain of *Aspergillus,* in particular *A. aculeatus, A. niger*, *A. awamori*, or *A. tubigensis*. The xylanase may be derived from a strain of *Trichoderma*, e.g., *T. reesei*, or from a strain of *Humicola*, e.g., *H. insolens.*

The xylanase may be derived from *Bacillus*, e.g., *B. halodurans* or *B.subtilis.*

Suitable commercially available xylanase preparations for use in the present invention include PANZEA BG™, PENTOPAN MONO BG™, PENTOPAN 500 BG™, PENTOPAN PLUS™, Panzea Dual™, and Fungamyl Super MA™ (available from Novozymes A/S), GRINDAMYL POWERBAKE™ (available from Danisco), and BAKEZYME BXP 5000™ and BAKEZYME BXP 5001™ (available from DSM).

## Dough

A method for preparing dough or a baked product prepared from the dough is disclosed which method comprises incorporating into the dough a glycosyltransferase, a cyclomaltodextrin glucanotransferase, or a mixture of a glycosyltransferase and a cyclomaltodextrin glucanotransferase.

Disclosed is further a dough comprising flour, water, and an effective amount of a baking composition or a premix according to the disclosure.

Disclosed are also methods for preparing a dough or a baked product comprising incorporating into the dough an effective amount of a baking composition of the present disclosure which improves one or more properties of the dough or the baked product obtained from the dough relative to a dough or a baked product in which a glycosyltransferase, a cyclomaltodextrin glucanotransferase, or a mixture of a glycosyltransferase and a cyclomaltodextrin glucanotransferase is not incorporated.

The phrase "incorporating into the dough" is defined herein as adding the baking composition according to the disclosure to the dough, to any ingredient from which the dough is to be made, and/or to any mixture of dough ingredients from which the dough is to be made. In other words, the baking composition of the disclosure may be added in any step of the dough preparation and may be added in one, two or more steps. The composition is added to the other dough ingredients that is kneaded and baked to make the baked product, using methods well known in the art.

The term "effective amount" is defined herein as an amount of baking composition according to the disclosure that is sufficient for providing a measurable effect on at least one property of interest of the dough and/or baked product.

The term "dough" is defined herein as a mixture of flour and other ingredients firm enough to knead or roll.

The dough of the disclosure may comprise flour derived from any cereal grain, including wheat, barley, rye, oat, corn, sorghum, rice, and millet, and any mixtures thereof. The dough may be with or without fibers from cereal grains.

The dough may also comprise other conventional dough ingredients, e.g., proteins, such as milk powder, gluten, and soy; eggs (either whole eggs, egg yolks or egg whites); an oxidant such as ascorbic acid, potassium bromate, potassium iodate, azodicarbonamide (ADA) or ammonium persulfate; an amino acid such as L-cysteine; a sugar; a salt such as sodium chloride, calcium acetate, sodium sulfate or calcium sulfate; an oil; shortening; fat; and calcium propionate.

The dough may comprise one or more emulsifiers selected from the group consisting of diacetyl tartaric acid esters of monoglycerides (DATEM), sodium stearoyl lactylate (SSL), calcium stearoyl lactylate (CSL), ethoxylated mono- and diglycerides (EMG), polysorbates (PS), succinylated monoglycerides (SMG), monoacylglyceroles (MAG), diacylglyceroles (DAG), and mixtures thereof. The amount of emulsifier in the dough may typically be between 0.01% (w/w) and 0.5% (w/w). Preferably, the amount of emulsifier in the dough is between 0.05% and 0.2%, such as around 0.1%.

The dough of the disclosure may be fresh, frozen or par-baked (pre-baked).

The dough of the disclosure is normally leavened dough or dough to be subjected to leavening. The dough may be leavened in various ways, such as by adding chemical leavening agents, e.g., sodium bicarbonate or by adding a leaven (fermenting dough), but it is preferred to leaven the dough by adding a suitable yeast culture, such as a culture of *Saccharomyces cerevisiae* (baker's yeast), e.g., a commercially available strain of *S. cerevisiae.*

The amount of glycosyltransferase may be 0.01-100 mg enzyme protein per kg flour in the dough, in particular 0.01-50 mg enzyme protein per kg flour, in particular 0.01-10 mg enzyme protein per kg flour, in particular 0.01-5 mg enzyme protein per kg flour, in particular 0.01-1 mg enzyme protein per kg flour.

The amount of cyclomaltodextrin glucanotransferase may be 0.01-100 mg enzyme protein per kg flour in the dough, in particular 0.01-50 mg enzyme protein per kg flour, in particular 0.01-10 mg enzyme protein per kg flour, in particular 0.01-5 mg enzyme protein per kg flour, in particular 0.01-1 mg enzyme protein per kg flour.

**Baked product**

The process of the invention may be used for any kind of baked product prepared from dough, either of a white, light or dark type. As used herein, "baked product" means any kind of baked product (in particular white, whole-meal or rye bread), including bread types such as pan bread, sandwich bread, toast bread, lidded toast bread, open bread, pan bread with and without lid, and any variety thereof; especially lidded toast bread.

**Slicing at high temperatures**

Core temperature: The core temperature of the baked product may be measured using a precision core thermometer (TFX 410 handheld thermometer, Ebro, Germany) fitted with a PT 1000 pointed probe by inserting the probe into the bread core immediately after retrieving bread from oven.

Bread was let to cool off on a perforated baking sheet kept at room temperature of e.g. (24 $\pm$ 0.5 °C) and held up in a rack by its edges so that hot air could move away from the bread. Bread was sliced when desired core bread temperature ($\pm$ 0.5 °C) or, e.g., ($\pm$ 1.0 °C) was reached.

According to the present invention, the baked product is sliced at a core temperature of the baked product of 30-55°C; e.g., at a core temperature of the baked product of 31-55°C; e.g., at a core temperature of the baked product of 32-55°C; e.g.; at a core temperature of the baked product of 33-55°C; e.g., at a core temperature of the baked product of 34-55°C; e.g., at a core temperature of the baked product of 35-55°C; e.g., e.g., at a core temperature of the baked product of 36-55°C; e.g., at a core temperature of the baked product of 37-55°C; e.g., at a core temperature of the baked product of 38-55°C; e.g., at a core temperature of the baked product of 39-55°C; e.g., at a core temperature of the baked product of 40-55°C; e.g., at a core temperature of the baked product of 41-55°C; e.g., at a core temperature of the baked product of 42-55°C; e.g., at a core temperature of the baked product of 43-55°C; e.g., at a core temperature of the baked product of 44-55°C; e.g., at a core temperature of the baked product of 45-55°C; e.g.; at a core temperature of the baked product of 35-38°C; e.g., at a core temperature of the baked product of 38-40°C; e.g., at a core temperature of the baked product of 40-42°C; e.g., at a core temperature of the baked product of 42-44°C; e.g., at a core temperature of the baked product of 44-46°C; e.g., at a core temperature of the baked product of 48-50°C; e.g., at a core temperature of the baked product of 50-55°C; e.g., at a core temperature of the baked product of 30-50°C; e.g., at a core temperature of the baked product of 35-50°C; e.g., at a core temperature of the baked product of 40-50°C.

The present invention is further described by the following examples which should not be construed as limiting the scope of the invention.

Example 1

Preparation of lidded toast bread

Lidded toast bread (700 g) was made from dough prepared according to a standard European straight dough procedure with 580 g water, 40 g yeast, 20 g salt, 20 g sugar, 60 ppm ascorbic acid, and 4 g calcium propionate (as preservative) per kg of flour.

Dough was scaled to 700 g, proofed (60 min, 36-38 °C, 80-90% relative humidity) and baked for 30 min at 225 °C in lidded pans.

The breads were baked in single (non-attached) baking pans in a rotary oven.

The breads were sliced at a core temperature of 48-50°C, using an automatic bread slicer, most preferably the Roto-Shop de Luxe slicer (Rego Hertlitzius Gmbh, Nordrhein-Westfalen, Germany). The slices had a thickness of approximately 12.5 mm.

All breads contained a common background of ascorbic acid (60 mg per kg flour), and enzymes to ensure that the bread had quality properties comparable to those found in commercial breads. The common background of enzymes was composed of fungal alpha-amylase (FUNGAMYL 2500 BG) at a dosage of 6 mg per kg flour, and xylanase (PANZEA BG) at a dosage of 30 mg per kg flour.

Example 2

Impact of glycosyltransferase (SEQ ID NO: 1) on bread sliceability

Lidded toast breads (700 g) were made according to Example 1 (control).

Lidded toast breads (700 g) were made according to Example 1 (control) except that additionally, 0.5% (w/w) Puratos' Soft'R Intense Sliceability™ was added (a commercial product from Puratos). Lidded toast breads (700 g) were made according to Example 1 (control) except that additionally, glycosyltransferase (SEQ ID NO:1) was added in an amount of 0.16 mg enzyme protein per kg flour.

The breads baked with glycosyltransferase (SEQ ID NO:1) showed slicing properties that were much better than the control, and also better than the breads baked with the commercial improver Puratos' Soft'R Intense Sliceability.

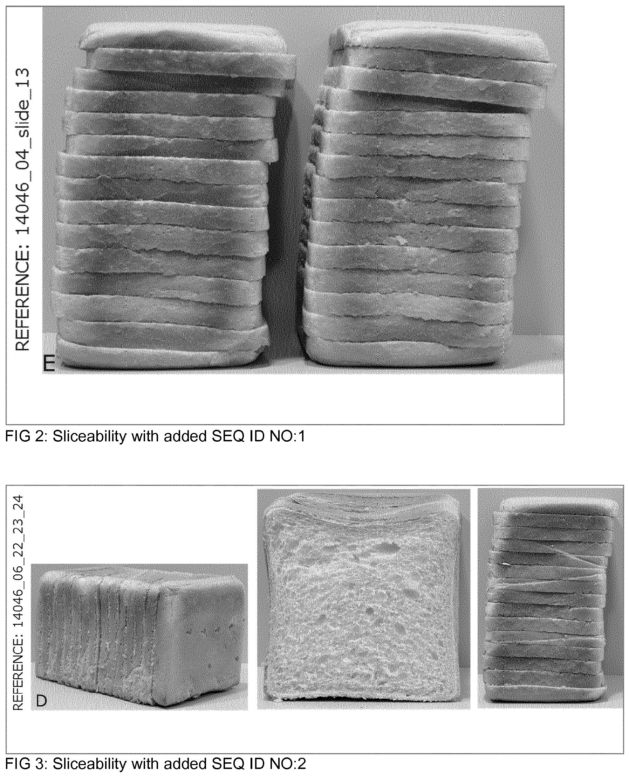

FIG 2 shows the bread baked with SEQ ID:1.

Example 3

Impact of cyclomaltodextrin glucanotransferase (SEQ ID NO: 2) on bread sliceability

Lidded toast breads (700 g) were made according to Example 1 except that additionally, cyclomaltodextrin glucanotransferase (SEQ ID NO:2) was added in an amount of 0.75 mg enzyme protein per kg flour.

The breads baked with cyclomaltodextrin glucanotransferase (SEQ ID NO: 2) showed improved sliceability as judged by reduced occurrence of loaf deformation, single or multiple slice deformation, and peeling of the crumb surface, no sticking of contiguous slices, reduced peeling off of crumb surface, and reduced overall deterioration of crumb grain texture.

FIG 3 shows the bread baked with SEQ ID NO:2.

Example 4

Impact of a mixture of glycosyltransferase (SEQ ID NO: 1) and cyclomaltodextrin glucanotransferase (SEQ ID NO: 2) on bread sliceability

Lidded toast breads (700 g) were made according to Example 1 (control).

Lidded toast bread (700 g) were made according to Example 1 except that additionally, 0.5% (w/w) Puratos' Soft'R Intense Sliceability™ was added (a commercial product from Puratos).

Lidded toast breads (700 g) were made according to Example 1 except that additionally, glycosyltransferase (SEQ ID NO:1) was added in an amount of 0.08 mg enzyme protein/kg flour and cyclomaltodextrin glucanotransferase (SEQ ID NO: 2) was added in an amount of 0.15 mg enzyme protein/kg flour.

FIG 1 shows the results.

The arrows show slicing defects such as the permanent deformation of single or multiple slices, peeling off of crumb surface, and undue deterioration of crumb grain texture.

Fig. 1 shows how slicing defects in loaf bread were improved by the use of glycosyltransferase (SEQ ID NO:1) and cyclomaltodextrin glucanotransferase (SEQ ID NO: 2) in combination, as judged by occurrence of loaf deformation, single or multiple slice deformation and peeling of the crumb surface, reduced overall deterioration of crumb grain texture.

Example 5

Impact of glycosyltransferase (SEQ ID NO: 1), and a mixture of glycosyltransferase (SEQ ID NO: 1) and cyclomaltodextrin glucanotransferase (SEQ ID NO: 2) on sliceability of lidded toast bread.

White pans breads were made in the following way (the flour used was Meneba Kolibri):

*Table 1. White pan bread recipe*

| Recipe: | % | g |
|---|---|---|
| Water | 59 | 1180 |
| Flour | 100 | 2000 |
| Yeast | 3.5 | 70 |
| Salt | 2 | 40 |
| Sugar | 2 | 40 |
| Calcium propionate | 0.4 | 8.0 |

### Table 2. Bread making process

| Process: | Time (min) |
|---|---|
| Spiral mixer slow | 0:02 |
| Spiral mixer fast | 0:10 |
| Dough evaluation | 0:05 |
| Scale 4 X 700g rounding | 0:05 |
| Table resting at 24°C (covered) | 0:10 |
| Moulding: 5/4/20/15 | 0:02 |
| Panning with 4piece method | 0:08 |
| Fermentation: 38°C; 90%RH | 1:00 |
| Baking rotary oven: 225°C/220°C | 0:30 |

All breads contained a common background of ascorbic acid (60 mg per kg flour), and enzymes to ensure that the bread had quality properties comparable to those found in commercial breads. The common background of enzymes was composed of fungal alpha-amylase (FUNGAMYL 2500 BG) at a dosage of 6 mg per kg flour, and xylanase (PANZEA BG) at a dosage of 30 mg per kg flour.

Additionally, either glycosyltransferase (SEQ ID NO:1) at a concentration of 0.10 mg enzyme protein per kg flour, or a mixture of glycosyltransferase (SEQ ID NO:1) at a concentration of 0.075 mg enzyme protein per kg flour and cyclomaltodextrin glucanotransferase (SEQ ID NO: 2) at a concentration of 0.12 mg enzyme protein per kg flour were added.

The bread loaves were sliced as described in Example 1. Bread loaves were sliced at a temperature of 44 ± 1 °C. Slice thickness was 12.5 mm.

The number of intact and damaged slices per loaf of bread was manually counted after the slicing step. The experiment was repeated four times (true replicates) and each replication value was the averaged count of four loaves of bread.

As shown in Table 3, the average number of intact slices in a loaf of bread increased and the number of damaged slices decreased due to addition of glycosyltransferase (SEQ ID NO:1), or the addition of glycosyltransferase (SEQ ID NO:1) and cyclomaltodextrin glucanotransferase (SEQ ID NO: 2). Table 4 shows the same effects as shown in Table 3 but expressed as a percentage of the total number of slices per loaf.

Table 3:

| Means of sliceability characteristics (# intact and # damaged slices per loaf of 15 slices) of bread as a function of enzyme treatment | | |
|---|---|---|
| Enzyme treatment | # Intact slices | #Damaged slices |
| No enzyme | 10.9 | 4.2 |
| glycosyltransferase (SEQ ID NO:1) in an amount of 0.10 mg enzyme protein per kg flour | 12.6 | 2.4 |
| glycosyltransferase (SEQ ID NO:1 ) was added in an amount of 0.075 mg enzyme protein/kg flour and cyclomaltodextrin glucanotransferase (SEQ ID NO: 2) was added in an amount of 0.12 mg enzyme protein/kg flour | 13.7 | 1.3 |

Table 4:

| Means of sliceability characteristics (percent intact and percent damaged slices of total slices in a loaf) of bread as a function of enzyme treatment | | |
|---|---|---|
| Enzyme treatment | # Intact slices | #Damaged slices |
| No enzyme | 72.9% | 27.9% |
| glycosyltransferase (SEQ ID NO:1) was added in an amount of 0.10 mg enzyme protein per kg flour | 84.2% | 15.8% |

(continued)

| Means of sliceability characteristics (percent intact and percent damaged slices of total slices in a loaf) of bread as a function of enzyme treatment | | |
|---|---|---|
| Enzyme treatment | # Intact slices | #Damaged slices |
| glycosyltransferase (SEQ ID NO:1 ) was added in an amount of 0.075 mg enzyme protein/kg flour and cyclomaltodextrin glucanotransferase (SEQ ID NO: 2) was added in an amount of 0.12 mg enzyme protein/kg flour | 91.3% | 8.8% |

Conclusion:

Table 3 and Table 4 clearly show that it is possible to increase the number of intact slices in a loaf by using the enzyme(s) of the present invention.

SEQUENCE LISTING

<110> Novozymes A/S

<120> METHOD TO IMPROVE SLICEABILITY OF BAKED GOODS

<130> 13067-WO-PCT

<160> 2

<170> PatentIn version 3.5

<210> 1
<211> 621
<212> PRT
<213> Rhodothermus obamensis

<400> 1

```
Met Ser Trp Leu Thr Glu Glu Asp Ile Arg Arg Trp Glu Ser Gly Thr
1               5                   10              15

Phe Tyr Asp Ser Tyr Arg Lys Leu Gly Ala His Pro Asp Asp Glu Gly
            20              25              30

Thr Trp Phe Cys Val Trp Ala Pro His Ala Asp Gly Val Ser Val Leu
        35              40              45

Gly Ala Phe Asn Asp Trp Asn Pro Glu Ala Asn Pro Leu Glu Arg Tyr
    50              55              60

Gly Gly Gly Leu Trp Ala Gly Tyr Val Pro Gly Ala Arg Pro Gly His
65              70              75              80

Thr Tyr Lys Tyr Arg Ile Arg His Gly Phe Tyr Gln Ala Asp Lys Thr
            85              90              95

Asp Pro Tyr Ala Phe Ala Met Glu Pro Pro Thr Gly Ser Pro Ile Glu
            100             105             110

Gly Leu Ala Ser Ile Ile Thr Arg Leu Asp Tyr Thr Trp His Asp Asp
        115             120             125

Glu Trp Met Arg Arg Arg Lys Gly Pro Ala Ser Leu Tyr Glu Pro Val
    130             135             140

Ser Ile Tyr Glu Val His Leu Gly Ser Trp Arg His Lys Arg Pro Gly
145             150             155             160

Glu Ser Phe Ser Tyr Arg Glu Ile Ala Glu Pro Leu Ala Asp Tyr Val
            165             170             175
```

```
Gln Glu Met Gly Phe Thr His Val Glu Leu Leu Pro Val Met Glu His
            180                 185                 190

Pro Tyr Tyr Gly Ser Trp Gly Tyr Gln Val Val Gly Tyr Tyr Ala Pro
            195                 200                 205

Thr Phe Arg Tyr Gly Ser Pro Gln Asp Leu Met Tyr Leu Ile Asp Tyr
    210                 215                 220

Leu His Gln Arg Gly Ile Gly Val Ile Leu Asp Trp Val Pro Ser His
225                 230                 235                 240

Phe Ala Ala Asp Pro Gln Gly Leu Val Phe Phe Asp Gly Thr Thr Leu
            245                 250                 255

Phe Glu Tyr Asp Asp Pro Lys Met Arg Tyr His Pro Asp Trp Gly Thr
            260                 265                 270

Tyr Val Phe Asp Tyr Asn Lys Pro Gly Val Arg Asn Phe Leu Ile Ser
            275                 280                 285

Asn Ala Leu Phe Trp Leu Glu Lys Tyr His Val Asp Gly Leu Arg Val
    290                 295                 300

Asp Ala Val Ala Ser Met Leu Tyr Arg Asp Tyr Ser Arg Lys Glu Trp
305                 310                 315                 320

Thr Pro Asn Ile Phe Gly Gly Arg Glu Asn Leu Glu Ala Ile Asp Phe
            325                 330                 335

Ile Lys Lys Phe Asn Glu Thr Val Tyr Leu His Phe Pro Glu Ala Met
            340                 345                 350

Thr Ile Ala Glu Glu Ser Thr Ala Trp Pro Gly Val Ser Ala Pro Thr
    355                 360                 365

Tyr Asn Asn Gly Leu Gly Phe Leu Tyr Lys Trp Asn Met Gly Trp Met
    370                 375                 380

His Asp Thr Leu Asp Tyr Ile Gln Arg Asp Pro Ile Tyr Arg Lys Tyr
385                 390                 395                 400

His His Asp Glu Leu Thr Phe Ser Leu Trp Tyr Ala Phe Ser Glu His
            405                 410                 415

Tyr Val Leu Pro Leu Ser His Asp Glu Val Val His Gly Lys Gly Ser
            420                 425                 430
```

```
Leu Trp Gly Lys Met Pro Gly Asp Asp Trp Gln Lys Ala Ala Asn Leu
        435             440             445

Arg Leu Leu Phe Gly His Met Trp Gly His Pro Gly Lys Lys Leu Leu
        450             455             460

Phe Met Gly Gly Glu Phe Gly Gln His His Glu Trp Asn His Asp Thr
465             470             475             480

Gln Leu Glu Trp His Leu Leu Asp Gln Pro Tyr His Arg Gly Ile Gln
                485             490             495

Leu Trp Val Cys Asp Leu Asn His Leu Tyr Arg Thr Asn Pro Ala Leu
            500             505             510

Trp His Asp Gly Pro Glu Gly Phe Glu Trp Ile Asp Phe Ser Asp Arg
        515             520             525

Asp Gln Ser Val Ile Cys Tyr Leu Arg Lys Asn Ala Gly Arg Met Leu
    530             535             540

Leu Phe Val Leu Asn Phe Thr Pro Val Pro Arg Glu His Tyr Arg Val
545             550             555             560

Gly Val Pro Ile Gly Gly Pro Trp His Glu Val Leu Asn Ser Asp Ala
            565             570             575

Val Ala Tyr Gly Gly Ser Gly Met Gly Asn Phe Gly Arg Val Glu Ala
        580             585             590

Val Pro Glu Ser Trp His Gly Arg Pro Phe His Leu Glu Leu Thr Leu
    595             600             605

Pro Pro Leu Ala Ala Leu Ile Leu Glu Pro Glu His Gly
    610             615             620
```

<210> 2
<211> 683
<212> PRT
<213> Thermoanaerobacter sp.

<400> 2

```
Ala Pro Asp Thr Ser Val Ser Asn Val Val Asn Tyr Ser Thr Asp Val
1               5               10              15

Ile Tyr Gln Ile Val Thr Asp Arg Phe Leu Asp Gly Asn Pro Ser Asn
            20              25              30
```

Asn Pro Thr Gly Asp Leu Tyr Asp Pro Thr His Thr Ser Leu Lys Lys
35                    40              45

Tyr Phe Gly Gly Asp Trp Gln Gly Ile Ile Asn Lys Ile Asn Asp Gly
50                    55              60

Tyr Leu Thr Gly Met Gly Ile Thr Ala Ile Trp Ile Ser Gln Pro Val
65          70              75                  80

Glu Asn Ile Tyr Ala Val Leu Pro Asp Ser Thr Phe Gly Gly Ser Thr
85                  90                  95

Ser Tyr His Gly Tyr Trp Ala Arg Asp Phe Lys Lys Thr Asn Pro Phe
100              105              110

Phe Gly Ser Phe Thr Asp Phe Gln Asn Leu Ile Ala Thr Ala His Ala
115              120              125

His Asn Ile Lys Val Ile Ile Asp Phe Ala Pro Asn His Thr Ser Pro
130              135              140

Ala Ser Glu Thr Asp Pro Thr Tyr Gly Glu Asn Gly Arg Leu Tyr Asp
145              150              155              160

Asn Gly Val Leu Leu Gly Gly Tyr Thr Asn Asp Thr Asn Gly Tyr Phe
165              170              175

His His Tyr Gly Gly Thr Asn Phe Ser Ser Tyr Glu Asp Gly Ile Tyr
180              185              190

Arg Asn Leu Phe Asp Leu Ala Asp Leu Asp Gln Gln Asn Ser Thr Ile
195              200              205

Asp Ser Tyr Leu Lys Ala Ala Ile Lys Leu Trp Leu Asp Met Gly Ile
210              215              220

Asp Gly Ile Arg Met Asp Ala Val Lys His Met Ala Phe Gly Trp Gln
225              230              235              240

Lys Asn Phe Met Asp Ser Ile Leu Ser Tyr Arg Pro Val Phe Thr Phe
245              250              255

Gly Glu Trp Tyr Leu Gly Thr Asn Glu Val Asp Pro Asn Asn Thr Tyr
260              265              270

Phe Ala Asn Glu Ser Gly Met Ser Leu Leu Asp Phe Arg Phe Ala Gln
275              280              285

```
Lys Val Arg Gln Val Phe Arg Asp Asn Thr Asp Thr Met Tyr Gly Leu
    290             295             300

Asp Ser Met Ile Gln Ser Thr Ala Ala Asp Tyr Asn Phe Ile Asn Asp
305             310             315             320

Met Val Thr Phe Ile Asp Asn His Asp Met Asp Arg Phe Tyr Thr Gly
                325             330             335

Gly Ser Thr Arg Pro Val Glu Gln Ala Leu Ala Phe Thr Leu Thr Ser
            340             345             350

Arg Gly Val Pro Ala Ile Tyr Tyr Gly Thr Glu Gln Tyr Met Thr Gly
            355             360             365

Asn Gly Asp Pro Tyr Asn Arg Ala Met Met Thr Ser Phe Asp Thr Thr
    370             375             380

Thr Thr Ala Tyr Asn Val Ile Lys Lys Leu Ala Pro Leu Arg Lys Ser
385             390             395             400

Asn Pro Ala Ile Ala Tyr Gly Thr Gln Lys Gln Arg Trp Ile Asn Asn
            405             410             415

Asp Val Tyr Ile Tyr Glu Arg Gln Phe Gly Asn Asn Val Ala Leu Val
            420             425             430

Ala Ile Asn Arg Asn Leu Ser Thr Ser Tyr Tyr Ile Thr Gly Leu Tyr
    435             440             445

Thr Ala Leu Pro Ala Gly Thr Tyr Ser Asp Met Leu Gly Gly Leu Leu
    450             455             460

Asn Gly Ser Ser Ile Thr Val Ser Ser Asn Gly Ser Val Thr Pro Phe
465             470             475             480

Thr Leu Ala Pro Gly Glu Val Ala Val Trp Gln Tyr Val Ser Thr Thr
            485             490             495

Asn Pro Pro Leu Ile Gly His Val Gly Pro Thr Met Thr Lys Ala Gly
            500             505             510

Gln Thr Ile Thr Ile Asp Gly Arg Gly Phe Gly Thr Thr Ala Gly Gln
    515             520             525

Val Leu Phe Gly Thr Thr Pro Ala Thr Ile Val Ser Trp Glu Asp Thr
```

<pre>
                530                    535                      540

        Glu Val Lys Val Lys Val Pro Ala Leu Thr Pro Gly Lys Tyr Asn Ile
        545                 550                 555                 560


        Thr Leu Lys Thr Ala Ser Gly Val Thr Ser Asn Ser Tyr Asn Asn Ile
                        565                 570                 575


        Asn Val Leu Thr Gly Asn Gln Val Cys Val Arg Phe Val Val Asn Asn
                        580                 585                 590


        Ala Thr Thr Val Trp Gly Glu Asn Val Tyr Leu Thr Gly Asn Val Ala
                        595                 600                 605


        Glu Leu Gly Asn Trp Asp Thr Ser Lys Ala Ile Gly Pro Met Phe Asn
                610                 615                 620


        Gln Val Val Tyr Gln Tyr Pro Thr Trp Tyr Tyr Asp Val Ser Val Pro
        625                 630                 635                 640


        Ala Gly Thr Thr Ile Glu Phe Lys Phe Ile Lys Lys Asn Gly Ser Thr
                        645                 650                 655


        Val Thr Trp Glu Gly Gly Tyr Asn His Val Tyr Thr Thr Pro Thr Ser
                        660                 665                 670


        Gly Thr Ala Thr Val Ile Val Asp Trp Gln Pro
                        675                 680
</pre>

**Claims**

1. A method of improving sliceability of a baked product prepared from dough comprising:

   - incorporating into the dough a glycosyltransferase (EC 2.4.1.18) and/or a cyclomaltodextrin glucanotransferase (EC 2.4.1.19);
   - baking the dough into a baked product; and
   - slicing the baked product during the cooling period when the baked product has a core temperature of 30-55°C.

2. The method according to claim 1, wherein the glycosyltransferase has at least 80% sequence identity to the polypeptide of SEQ ID NO: 1.

3. The method according to any of the preceding claims, wherein the cyclomaltodextrin glucanotransferase has at least 80% sequence identity to the polypeptide of SEQ ID NO: 2.

4. The method according to any of the preceding claims, wherein a glycosyltransferase and a cyclomaltodextrin glucanotransferase are incorporated into the dough.

5. The method according to any of the preceding claims, which further comprises adding to the dough an enzyme selected from the group consisting of amylase, xylanase, glucanase, galactanase, mannanase, aminopeptidase, alpha-amylase, beta-amylase, anti-staling alpha-amylase, carboxypeptidase, catalase, chitinase, cutinase, deoxyribonuclease, esterase, alpha-galactosidase, beta-galactosidase, glucoamylase, alpha-glucosidase, beta-glucosidase, haloperoxidase, invertase, laccase, lipase, phospholipase, mannosidase, oxidase, pectinolytic enzymes, peptidoglutaminase, protease, peroxidase, phytase, and polyphenoloxidase.

6. The method according to any of the preceding claims, wherein the glycosyltransferase and/or the the cyclomaltodextrin glucanotransferase is applied in an amount of 0.01-100 mg enzyme protein per kg flour.

7. The method according to any of the preceding claims, wherein the baked product is sliced at a core temperature of the baked product of 38-55°C.

8. The method according to any of the preceding claims, wherein the sliceability of the baked product is improved compared to a baked product prepared from dough wherein no glycosyltransferase and/or a cyclomaltodextrin glucanotransferase is added to the dough.

9. The method according to any of the preceding claims, wherein the baked product is a bread; preferably a toast bread; in particular a lidded toast bread.

10. The method according to any of the preceding claims, wherein the crumb structure of the baked product is improved compared to a baked product prepared from dough wherein no glycosyltransferase and/or a cyclomaltodextrin glucanotransferase is added to the dough.

**Patentansprüche**

1. Verfahren zur Verbesserung der Schneidbarkeit von Backwaren, die aus Teig hergestellt werden, umfassend:

   - Einbringen einer Glycosyltransferase (EC 2.4.1.18) und/oder einer Cyclomaltodextringlucanotransferase in den Teig;
   - Backen des Teigs zu einer Backware; und
   - Schneiden der Backware während des Abkühlzeitraums, wenn die Backware eine Kerntemperatur von 30 bis 55 °C aufweist.

2. Verfahren nach Anspruch 1, wobei die Glycosyltransferase mindestens 80 % Sequenzidentität mit dem Polypeptid der SEQ ID NO: 1 aufweist.

3. Verfahren nach einem der vorhergehenden Ansprüche, wobei die Cyclomaltodextringlucanotransferase mindestens 80 % Sequenzidentität mit dem Polypeptid der SEQ ID NO: 2 aufweist.

4. Verfahren nach einem der vorhergehenden Ansprüche, wobei eine Glycosyltransferase und eine Cyclomaltodextringlucanotransferase in den Teig eingebracht werden.

5. Verfahren nach einem der vorhergehenden Ansprüche, ferner umfassend das Hinzugeben eines Enzyms zu dem Teig, ausgewählt aus der Gruppe bestehend aus Amylase, Xylanase, Glucanase, Galactanase, Mannanase, Aminopeptidase, Alpha-Amylase, Beta-Amylase, Haltbarkeitszusatz Alpha-Amylase, Carboxypeptidase, Catalase, Chitinase, Cutinase, Desoxyribonuklease, Esterase, Alpha-Galactosidase, Beta-Galactosidase, Glucoamylase, Alpha-Glucosidase, Beta-Glucosidase, Halogenperoxidase, Invertase, Laccase, Lipase, Phospholipase, Mannosidase, Oxidase, pectinolytische Enzyme, Peptidoglutaminase, Protease, Peroxidase, Phytase und Polyphenoloxidase.

6. Verfahren nach einem der vorhergehenden Ansprüche, wobei die Glycosyltransferase und/oder die Cyclomaltodextringlucanotransferase in einer Menge von 0,01 bis 100 mg Enzymprotein je kg Mehl angewandt wird.

7. Verfahren nach einem der vorhergehenden Ansprüche, wobei die Backware bei einer Kerntemperatur der Backware von 38 bis 55 °C geschnitten wird.

8. Verfahren nach einem der vorhergehenden Ansprüche, wobei die Schneidbarkeit der Backware im Vergleich zu einer Backware, die aus Teig hergestellt wird, wobei keine Glycosyltransferase und/oder eine Cyclomaltodextringlucanotransferase zu dem Teig hinzugefügt wird, verbessert ist.

**9.** Verfahren nach einem der vorhergehenden Ansprüche, wobei die Backware Brot ist; vorzugsweise ein Toastbrot; insbesondere ein Toastbrot aus einer Backform mit Deckel.

**10.** Verfahren nach einem der vorhergehenden Ansprüche, wobei die Krumenstruktur der Backware im Vergleich zu einer Backware, die aus Teig hergestellt wird, wobei keine Glycosyltransferase und/oder eine Cyclomaltodextringlucanotransferase zu dem Teig hinzugefügt wird, verbessert ist.

**Revendications**

**1.** Méthode d'amélioration de l'aptitude au tranchage d'un produit de panification préparé à partir d'une pâte, comprenant :

- l'incorporation dans la pâte d'une glycosyltransférase (EC 2.4.1.18) et/ou d'une cyclomaltodextrine glucanotransférase ;
- la cuisson de la pâte en un produit de panification ; et
- le tranchage du produit de panification pendant la période de refroidissement lorsque le produit de panification possède une température de coeur de 30-55 °C.

**2.** Méthode selon la revendication 1, dans laquelle la glycosyltransférase possède au moins 80 % d'identité de séquence avec le polypeptide de SEQ ID n° 1.

**3.** Méthode selon l'une quelconque des revendications précédentes, dans laquelle la cyclomaltodextrine glucanotransférase possède au moins 80 % d'identité de séquence avec le polypeptide de SEQ ID n° 2.

**4.** Méthode selon l'une quelconque des revendications précédentes, dans laquelle une glycosyltransférase et une cyclomaltodextrine glucanotransférase sont incorporées dans la pâte.

**5.** Méthode selon l'une quelconque des revendications précédentes, comprenant en outre l'addition à la pâte d'une enzyme choisie dans le groupe constitué par une amylase, une xylanase, une glucanase, une galactanase, une mannanase, une aminopeptidase, une alpha-amylase, une bêta-amylase, une alpha-amylase anti-rassissement, une carboxypeptidase, une catalase, une chitinase, une cutinase, une désoxyribonucléase, une estérase, une alpha-galactosidase, une bêta-galactosidase, une glucoamylase, une alpha-glucosidase, une bêta-glucosidase, une halopéroxydase, une invertase, une laccase, une lipase, une phospholipase, une mannosidase, une oxydase, les enzymes pectinolytiques, une peptidoglutaminase, une protéase, une peroxydase, une phytase, et une polyphénol oxydase.

**6.** Méthode selon l'une quelconque des revendications précédentes, dans laquelle la glycosyltransférase et/ou la cyclomaltodextrine glucanotransférase sont appliquées selon une quantité de 0,01-100 mg de protéine enzymatique par kg de farine.

**7.** Méthode selon l'une quelconque des revendications précédentes, dans laquelle le produit de panification est tranché à une température de coeur du produit de panification de 38-55 °C.

**8.** Méthode selon l'une quelconque des revendications précédentes, dans laquelle l'aptitude au tranchage du produit de panification est améliorée par rapport à un produit de panification préparé à partir d'une pâte où aucune glycosyltransférase et/ou cyclomaltodextrine glucanotransférase n'est ajoutée à la pâte.

**9.** Méthode selon l'une quelconque des revendications précédentes, dans laquelle le produit de panification est un pain ; préférablement un pain de mie ; en particulier un pain de mie préparé dans un moule à couvercle.

**10.** Méthode selon l'une quelconque des revendications précédentes, dans laquelle la structure de la mie du produit de panification est améliorée par rapport à un produit de panification préparé à partir d'une pâte où aucune glycosyltransférase et/ou cyclomaltodextrine glucanotransférase n'est ajoutée à la pâte.

FIG 1: Sliceability with a mixture of SEQ ID NO:1 and SEQ ID NO:2

FIG 2: Sliceability with added SEQ ID NO:1

FIG 3: Sliceability with added SEQ ID NO:2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070015679 A
- WO 9517413 A
- WO 9522625 A
- US 5223409 A
- WO 9206204 A
- WO 8903421 A
- WO 8402921 A
- WO 9826057 A
- WO 0032758 A

**Non-patent literature cited in the description**

- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **TAKATA et al.** *Applied and Environmental Microbiology,* 1994, 3097
- **H. NEURATH ; R.L. HILL.** In, The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **SMITH et al.** *J. Mol. Biol.,* 1992, vol. 224, 899-904
- **BOEL et al.** *EMBO J.,* 1984, vol. 3 (5), 1097-1102
- *Agric. Biol. Chem.,* 1991, vol. 55 (4), 941-949